(19) Europäisches Patentamt
European Patent Office
Office européen des brevets

(11) Publication number: **0 140 482**
**B1**

(12) # EUROPEAN PATENT SPECIFICATION

(45) Date of publication of patent specification: **13.01.88**

(51) Int. Cl.[4]: **C 07 C 51/58,** C 07 C 63/68

(21) Application number: **84305016.2**

(22) Date of filing: **24.07.84**

(54) **Fluorination process for the production of nuclear fluorinated phthaloyl- and terephthaloyl fluorides.**

(30) Priority: **16.09.83 GB 8324902**

(43) Date of publication of application:
**08.05.85 Bulletin 85/19**

(45) Publication of the grant of the patent:
**13.01.88 Bulletin 88/02**

(84) Designated Contracting States:
**BE CH DE FR GB IT LI NL**

(56) References cited:
**EP-A-0 010 651**
**EP-A-0 024 516**
**EP-A-0 038 223**

(73) Proprietor: **IMPERIAL CHEMICAL INDUSTRIES PLC**
**Imperial Chemical House Millbank**
**London SW1P 3JF (GB)**

(72) Inventor: **Cleare, Peter John Vernon**
**29 Gainsborough Drive**
**Ascot Berkshire SL5 8TA (GB)**
Inventor: **Costello, Alan Thomas**
**6 Knowle Avenue**
**Ashton-under-Lyne Lancashire (GB)**

(74) Representative: **Houghton, Malcolm John et al**
**Imperial Chemical Industries PLC**
**Legal Department: Patent PO Box 6**
**Welwyn Garden City Herts, AL7 1HD (GB)**

EP 0 140 482 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for the preparation of nuclear fluorinated phthaloyl and terephthaloyl fluorides which are useful chemical intermediates in the synthesis of pesticidal compounds.

A process for the preparation of tetrafluorophthaloyl fluoride is reported in Chem. Abs. 1966, *64*, 14125. It consists of heating tetrachlorophthaloyl chloride with potassium fluoride at 160°C for 12 hours and at 270°C for 8 hours in a steel autoclave to give tetrafluorophthaloyl fluoride in 20% yield. A similarly protracted, high temperature process for the corresponding terephthaloyl compound in which tetrachloroterephthaloyl chloride is heated in a stirred autoclave for 18 hours at 140°C and then for 34 hours at 270°C, is reported in Chem. Abs. 1966, *64*, 6532. The product, presumably tetrafluoroterephthaloyl fluoride, is extracted with methanol and isolated as the dimethyl ester of tetrafluoroterephthalic acid.

A process for the preparation of nuclear fluorinated benzoyl fluorides from corresponding nuclear chlorinated benzoyl halides is described in DE 3140634 A1. The process comprises reacting a nuclear chlorinated benzoyl halide with potassium fluoride in a diluent optionally in the presence of a catalyst and is characterised by operating at an elevated temperature and the boiling pressure of the reaction mixture and distilling off the nuclear fluorinated benzoyl fluoride during the reaction. As diluents, inert organic solvents having a lower vapour pressure than the product are mentioned. Tetramethylene sulphone is instanced for the fluorination of pentachlorobenzoyl chloride. Generally high temperatures in excess of 200°C are required and the desired product tends to be contaminated with partially fluorinated compounds.

According to the present invention there is provided a process for the preparation of a compound of the formula (I):

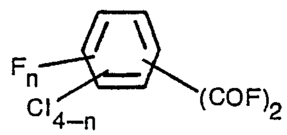

(I)

in which n is an integer of from 1 to 4, which comprises reacting a compound of the formula (II):

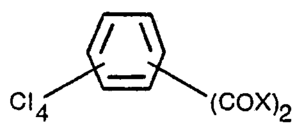

(II)

in which X is halogen, with potassium fluoride in a polar aprotic solvent at a temperature of from 100 to 200°C, preferably 120 to 150°C. The invention also includes the compound (I) whenever obtained by this process.

The carboxylic acid halide groups, which in compound (II) are conveniently carboxylic acid chloride groups, will normally be in positions on the benzene ring *ortho* or *para* to each other.

The process of the invention is of particular value for the preparation of compounds of formula (I) in which n is 4 and especially for the preparation of tetrafluoroterephthaloyl fluoride from tetrachloroterephthaloyl chloride. However, it is envisaged that the degree of nuclear fluorination may be varied by careful process control, e.g. by adjustment of the quantity of potassium fluoride used, the choice of solvent and/or temperature, such that the integer n has values of 1, 2 or 3.

Any polar aprotic solvent may be used, but some are better than others. For preparing tetrafluoroterephthaloyl fluoride from tetrachloroterephthaloyl chloride, sulpholane is much preferred in terms of yield and ease of recovery. Diphenyl sulphone is comparable as a reaction medium but, being a higher boiling solvent, recovery is more difficult. Nitrobenzene is satisfactory but dimethylacetamide, N-methyl pyrrolidone and benzonitrile are less effective. Dimethyl sulphoxide is not recommended as it can give explosive mixtures with acid halides. For preparing di- and tri-nuclear fluorinated products from tetrachloroterephthaloyl chloride, diglyme is preferred.

A catalyst, particularly a phase transfer catalyst, such as a quaternary ammonium halide, or a crown ether, may increase product yield but should be used with caution. Tetrabutyl ammonium bromide, for example, tends to react with product. When used with diglyme, a phase transfer catalyst can substantially increase the amount of nuclear fluorination.

The starting compounds, tetrachloroterephthaloyl chloride and tetrachlorophthaloyl chloride, are described in the chemical literature (Chem. Abs. 1965, *62*, 470 and Chem. Abs. 1966, *64*, 14125, respectively). The former is available commercially. Other halides which may be used as the starting compound (II), may be obtained from the tetrachlorobenzene dicarboxylic acid chlorides by techniques well known for converting acid chlorides to other acid halides. The preparation of, for example, tetrachloroterephthaloyl fluoride from tetrachloroterephthaloyl chloride is described in Chem. Abs. 1965, *62*, 470.

In carrying out the process of the invention, the potassium fluoride and solvent are preferably first dried by distilling off water with some of the solvent, or, if the boiling point of the solvent is too high, with a small added amount of a second solvent, such as toluene, which forms an azeotropic mixture with water. Conveniently, the compound (II) and optionally a catalyst are added to the potassium fluoride and heated together in an agitated reaction vessel. The temperature at which they are heated will depend on the

degree of nuclear fluorination required. As a guide, one or two chlorine atoms will be replaced by fluorine atoms over a temperature range of from 100 to 130°C, three chlorine atoms at temperatures above 130°C and four chlorine atoms at temperatures above 140°C. Chemical degradation is apparent at temperatures above 200°C, sometimes even at lower temperatures above 150°C. Typically, to replace four chlorine atoms by fluorine atoms, the ingredients are heated at 120° to 150°C for several hours. After reaction, the product may be isolated as compound (I) by vacuum distillation or more conveniently, as a diester of compound (I), such as the dimethyl ester, by quenching the reaction mixture in the appropriate alcohol, filtering to remove inorganic material, evaporating the filtrates under reduced pressure to remove excess alcohol and drowning into water to precipitate the diester.

The potassium fluoride may be used in stoichiometric amount or excess. No more than a 50% excess is necessary. Where all four nuclear-bound chlorine atoms are to be substituted by fluorine an excess of 30% or less is satisfactory. The addition of a small amount of caesium fluoride may prove beneficial.

The nuclear fluorinated phthaloyl and terephthaloyl fluorides obtained by the present invention are valuable chemical intermediates for the synthesis of pesticides via, for example, the corresponding methyl tetrafluorobenzyl alcohol. This may be derived from compound (I) by reduction with sodium borohydride in diglyme at room temperature, such as described in UK Patent Specification No. 2127013, followed by catalytic hydrogenation of the tetrafluoroxylyldialcohol so obtained.

The invention is illustrated by the following Examples 1 to 9.

### Example 1

Preparation of tetrafluoroterephthaloyl fluoride (TFTF) and isolation as the dimethyl ester of tetrafluoroterephthalic acid

Potassium fluoride (13 g) was stirred and heated in sulpholane (30 ml) to 200°C. Vacuum was applied to remove, by distillation, residual moisture and any other material present with a boiling point below 150°C at 15—20 mm Hg. The mixture was cooled to 20 to 30°C and tetrachloroterephthaloyl chloride (8.75 g) added. The temperature was raised to 150°C and the mixture stirred for 2—3 hours at this temperature to complete the reaction.

The product, which had been isolated in a previous similar preparation and identified as tetrafluoroterephthaloyl fluoride by comparison of its melting point and IR spectrum with those of an authentic sample, was isolated as the dimethyl ester by drowning the reaction mixture into methanol (200 ml) and filtering it to remove the inorganic material. The filtrates were then evaporated under reduced pressure to remove the excess methanol, and the residual material drowned into water (200 ml). The dimethyl ester precipitated as a grey solid and was collected by filtration. (Weight 4.2 g).

### Example 2

Preparation of TFTF in sulpholane at 130°C

Potassium fluoride (85.3 g) and sulpholane (441 g) were stirred and heated together under vacuum and 141 g of sulpholane removed by distillation in order to dry the system. The mixture was cooled to below 80°C and tetrachloroterephthaloyl chloride (67.5 g) added. The reaction temperature was raised to 130°C and maintained at this temperature for 12 hours. On completion of the reaction, the product tetrafluoroterephthaloyl fluoride, was isolated by distillation under vacuum at 20 mm of mercury. The fraction boiling between 110°C and 140°C was collected to give 46 g of material of approximately 80% strength.

### Example 3

Preparation of a mixture of dichloro-difluoro-, monochloro-trifluoro- and TFTF in diglyme at 150°C

Potassium fluoride (10.2 g) and diglyme (50 g) were stirred and heated together and 25 g of diglyme distilled off to dry the system. The mixture was cooled to below 80°C and tetrachloroterephthaloyl chloride (8.75 g) added. The reaction was stirred at 150°C for 21 hours to give a mixture containing 72% of the dichloro-difluoro-terephthaloyl fluoride, 22% of the monochloro-trifluoro-terephthaloyl fluoride and 6% of the tetrafluoroterephthaloyl fluoride.

### Example 4

Repeat of Example 3 but using a phase transfer catalyst at 120°C

The reaction in Example 3 was repeated in the presence of 0.5 g of tetrabutyl ammonium bromide at 120°C. After 14 hours a mixture consisting of 78% of tetrafluoroterephthaloyl fluoride, 11% of monochlorotrifluoroterephthaloyl fluoride, and 11% of dichlorodifluoroterephthaloyl fluoride was obtained.

### Example 5

Preparation of TFTF in diphenyl sulphone at 150°C

Potassium fluoride (10.2 g), diphenyl sulphone (25 g) and toluene (25 g) were stirred and heated and the system dried by removing the toluene by distillation. The reaction mixture was cooled and tetrachloroterephthaloyl chloride (8.75 g) added. The temperature was raised to 150°C and maintained at this temperature for 3 hours to complete the reaction. The product was isolated by distillation.

Example 6

Preparation of TFTF in dimethylacetamide at 120°C

Potassium fluoride (10.9 g) and dimethylacetamide (45 g) were stirred and heated. 15 g of solvent were removed by distillation to dry the system. The mixture was cooled to below 100°C and tetrachloroterephthaloyl chloride (9.35 g) added. The reaction temperature was then raised to 120°C and maintained at this temperature for 6 hours. The mixture was stirred and cooled to 25°C and methanol (30 g) added. This was drowned into water (250 g) and the solid collected by filtration to give 2.7 g of tetrafluorodimethyl-terephthalate.

Example 7

Preparation of TFTF in nitrobenzene at 130°C

Potassium fluoride (10.9 g) and nitrobenzene (45 g) were stirred and heated, under vacuum. 15 g of the solvent were removed by distillation at 50 mm of Hg in order to dry the system. The mixture was cooled to below 80°C and tetrachloroterephthaloyl chloride (9.53 g) added followed by 0.9 g of tetrabutylammonium bromide. The reaction temperature was raised to 130°C and maintained at this temperature for 20 hours to complete the reaction. The product was isolated as in Example 6 to give 3.8 g of tetrafluorodimethylterephthalate.

Example 8

Preparation of TFTF in N-methyl-2-pyrrolidone at 120°C

9.5 g of potassium fluoride and 45 g of N-methyl-2-pyrrolidone were stirred and heated and 15 g of the solvent removed by vacuum distillation to dry the system (NP 120°C at 50 mm of Hg). The mixture was cooled to below 60°C and 7.5 g of the tetrachloroterephthaloyl chloride added. The reaction temperature was raised to 120°C and maintained at this temperature for 6 hours to complete the reaction. The reaction mixture was then cooled to 25°C, 50 g of methanol added, and the mixture filtered. The filtrates were drowned into an excess of water to precipitate the product. The product was collected by filtration and dried to give 1.3 g of tetrafluorodimethylterephthalate.

Example 9

Preparation of TFTF in benzonitrile at 150°C

9.5 g of potassium fluoride and 45 g of benzonitrile were stirred and heated and 15 g of benzonitrile distilled from the reaction mixture under vacuum (BP 100°C at 50 mm of Hg) to dry the system. The mixture was cooled to below 60°C and 7.5 g of the tetrachloroterephthaloyl chloride added.

The reaction temperature was raised to 150°C and maintained at this temperature for 5 hours. G.C. examination of the reaction showed that the product was then cooled to 120°C and 0.8 g of tetrabutylammonium bromide added. The reaction temperature was then raised to 140°C and maintained at this temperature for 20 hours to complete the reaction. 1.9 g of the required product was isolated as the dimethyl ester.

**Claims**

1. A process for the preparation of a compound of the formula (I):

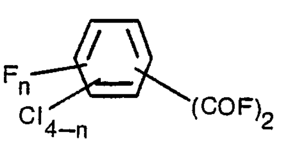

(I)

in which n is an integer of from 1 to 4, which comprises reacting a compound of the formula (II):

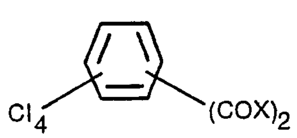

(II)

in which X is halogen, with potassium fluoride in a polar aprotic solvent at a temperature of from 100 to 200°C.

2. A process according to claim 1 in which the temperature is from 120 to 150°C.

3. A process according to claims 1 or 2 in which the polar aprotic solvent is sulpholane.

4. A process according to any one of the preceding claims in which compound (I) is tetrafluoroterephthaloyl fluoride and compound (II) is tetrachloroterephthaloyl chloride.

**Patentansprüche**

1. Verfahren zur Herstellung einer Verbindung der Formel (I)

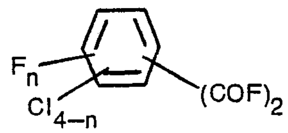

(I)

worin n für eine Ganzzahl von 1 bis 4 steht, bei welchem eine Verbindung der Formel (II)

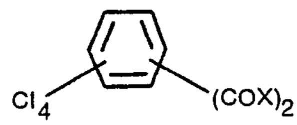

(II)

worin X für Halogen steht, mit Kaliumfluorid in einem polaren aprotischen Lösungsmittel bei einer Temperatur von 100 bis 200°C umgesetzt wird.

2. Verfahren nach Anspruch 1, bei welchem eine Temperatur von 120 bis 150°C verwendet wird.

3. Verfahren nach einem der Ansprüche 1 oder 2, bei welchem als polares aprotisches Lösungsmittel Sulfolan verwendet wird.

4. Verfahren nach einem der vorhergehenden Ansprüche, bei welchem die Verbindung (I) Tetrafluoroterephthaloylfluorid und die Verbindung (II) Tetrachloroterephthaloylchlorid ist.

**Revendications**

1. Procédé de préparation d'un composé de formule (I):

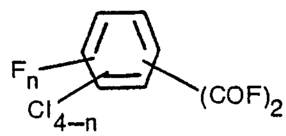

(I)

dans laquelle n est un nombre entier de 1 à 4, qui consiste à faire réagir un composé de formule (II):

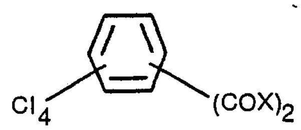

(II)

dans laquelle X est un halogène, avec le fluorure de potassium dans un solvant aprotique polaire à une température de 100 à 200°C.

2. Procédé suivant la revendication 1, dans lequel la température va de 120 à 150°C.

3. Procédé suivant la revendication 1 ou 2, dans lequel le solvant aprotique polaire est le sulfolane.

4. Procédé suivant l'une quelconque des revendications précédents, dans lequel le composé (I) est le fluorure de tétrafluorotéréphtaloyle et le composé (II) est le chlorure de tétrachlorotéréphtaloyle.